# EUROPEAN PATENT SPECIFICATION

(11) **EP 2 740 787 B1**
(45) Date of publication and mention of the grant of the patent: **09.12.2015**
(21) Application number: 12766109.8
(22) Date of filing: 30.07.2012
(51) Int. Cl.: C12M 1/00

(54) **PHOTOBIOREACTOR FOR CULTURING PHOTOAUTOTROPHIC MICROORGANISMS**
PHOTOBIOREAKTOR ZUR KULTUR PHOTOAUTOTROPHER MIKROORGANISMEN
PHOTOBIORÉACTEUR DESTINÉ À LA CULTURE DE MICROORGANISMES PHOTOAUTOTROPHES

(30) Priority: 01.08.2011 ES 201131337 P
(43) Date of publication of application: 11.06.2014
(73) Proprietor: Algaenergy S. A., 28108 Alcobendas (Madrid) (ES)
(72) Inventor: LLAMAS MOYA, Bernardo, E-28108 Alcobendas (Madrid) (ES); SEGURA FORNIELES, María, E-28108 Alcobendas (Madrid) (ES)
(74) Representative: Monzón de la Flor, Luis Miguel
(86) International application number: PCT/ES2012/070591
(87) International publication number: WO 2013/017723

(56) References cited:
- WO-A1-2011/036517
- WO-A2-2005/006838
- WO-A2-2009/155032
- GB-A- 2 339 763

## Description

### OBJECT OF THE INVENTION

The present invention is in the technical field of culturing photoautotrophic microorganisms such as microalgae or cyanobacteria with the aim of producing algal biomass, which can be used for production of biofuels and other purposes. Specifically, the purpose of the invention refers to a photobioreactor for culturing photoautotrophic microorganisms.

### BACKGROUND OF THE INVENTION

It is possible to use sunlight and water rich in nutrients for mass scale growth of photoautotrophic microorganisms such as microalgae, as efficient generators of various products with high industrial value.

There are two basic designs for the production of photoautotrophic microorganisms: open systems, in which the culture is continuously exposed to the atmosphere, and closed systems, commonly called photobioreactors, in which the culture has zero or very little contact with the atmosphere.

Open systems, also called raceway (circuit) type, are made up of shallow water channels (15 - 20 cm) in the form of a circuit, in which the culture medium is driven by rotating paddles. They generally occupy large surface areas (500 - 5000 m²), but have the advantage of low cost for the production of algal biomass in some specific geographical areas. However, the development of this technology seems to have reached its limit. Among the most serious drawbacks are the low productivity, easy contamination, costly recovery of product in dilute media and difficulty in controlling the temperature.

The drawbacks listed above have stimulated the development of closed photobioreactors constructed of transparent materials including glass and polycarbonate.

In closed tubular systems, the culture circulates through the tubes of a rigid material, either transparent or translucent. The circulation of the culture is carried out by impulsion, either with a pump or by an airlift system. The system comprises a sunlight capture module made of methacrylate or glass tubes, situated in parallel and connected together by "U" shaped pieces of the same or similar materials in the form of a coil.

In flat photobioreactors, the culture circulates in the interior of a compartment defined between parallel plates (sandwich type) of transparent material.

Flat vertical panel airlift-driven photobioreactors have a culture chamber comprising a series of elements made of vinyl polychloride or polyethylene terephthalate forming a serpentine course for the culture to traverse. With the aid of "static mixers", the microalgal cells are transported from the dark area of the reactor to the illuminated surface layer at fixed intervals. These photobioreactors can be of different sizes, the smallest of 5 litres, ideal for non-industrial laboratory use, intermediate of 33 litres, and the largest of 180 litres, created by Subitec (spin-off from the Fraunhofer IGB institute).

The photobioreactor called "ProviAPT vertical flat panel" from the company PROVIRON includes a plurality of parallel panels arranged in a battery, enclosed in water bags for regulating the temperature. It is made of recyclable thin film polypropylene and does not require additional supporting structures (Buehner et al. 2009).

The company Vertigro (USA) has a vertical photobioreactor for obtaining algae, made of parallel channel systems for the circulation of the culture and arranged vertically. The photobioreactor comprises receptacles in the form of polyethylene bags arranged inside a metal cage structure.

International patent application WO2009/155032 refers to a bioreactor with a channel in fluid connection with a container for absorbing CO₂ and making the microalgae grow. The bioreactor comprises two films of flexible material of some 150 micrometres thickness, facing each other and thermosealed along their edges to define a closed receptacle and also in the interior to define internal circulation channels.

International patent application WO2008/151376 describes a photobioreactor for continuous culture of photosynthetic organisms using artificial light, comprising at least a photobioreactor and a source of electromagnetic radiation, where said source is a diode that comprises an organic or polymeric emission film and where the material is manufactured in polymeric material (preferably polyethylene). The bioreactor includes inputs for water and culture medium in the top and outputs at the bottom. The equipment is contained in a structure that is opaque to sunlight.

In International patent application WO 2009155932 is disclosed a method and apparatus for growing algae for sequestering carbon dioxide and then harvesting the algae includes a container for a suspension of algae in a liquid and a bioreactor having a translucent channel in fluid communication with the container to absorb CO2 and grow the algae.

In WO2011036517 is disclosed a system and method for growing photosynthetic micro-organism. The system, comprises: a culture tank capable of retaining a micro-organism culture and the water, a cooling tank connected with the culture tank for controlling the temperature of the water of the culture tank; a holding tank capable of holding the micro-organism at a required temperature and light; and at least a bioreactor capable of providing maximum exposure of sunlight to the micro-organisms and maintaining continuous flow of the micro-organism.

In GB2339763 is disclosed a partitioned bag for use as a photo-bioreactor, for production of algae.

The microalgae production systems described above are costly, making the production of algal biomass expensive and setting a price that excludes its conversion into biofuel or for the production of any other low value products.

Therefore there is a need to find new designs and new alternatives that integrate high productivity and reduced cost, given that between 75 % and 85 % of the investment necessary for microalgae culture in the open is derived from the cost of the photobioreactor.

### DESCRIPTION OF THE INVENTION

The present invention resolves the technical and cost problems described above by means of a photobioreactor for culturing photoautotrophic microorganisms, such as microalgae and/or cyanobacteria, increasing culture efficiency and reducing production costs compared to the systems known in the state of the art, having the features of claim 1.

The photobioreactor of the invention comprises at least a growth enclosure adapted for allowing circulation of photoautotrophic microorganisms, enabling access of light to the culture, and a tank connected to this enclosure by means of some first pipes through which the culture circulates from the tank to the enclosure and some second pipes for returning the culture from the enclosure to the tank, in a closed circuit.

The enclosure is made of two films of flexible polymeric plastic material that can be transparent or translucent, fixed together by thermosealant, defining a plurality of parallel channels through which the culture circulates, these channels connecting to each other, constituting a single zigzag pipe. Materials suitable for the enclosure include, for example, vinyl polychloride, either high density and low density polyethylene, polystyrene, polypropylene, polyvinyl acetate and polyurethane, with low density polyethylene being particularly suitable due to is low cost.

The enclosure is suitable for leaving out in the open, so that the culture circulating within it is exposed to sunlight. The orientation of the enclosure is preferably that which enables the greatest access to sunlight during the period of operation. The use of sunlight implies energy savings and reduced operating costs of the photobioreactor, given that it is not necessary to provide equipment for artificial illumination (lamps). In any case, there is no problem with the enclosure operating inside and/or being irradiated by means of lamps.

In the description below, what is called the length of the enclosure will be the dimension of the longitudinal direction, that is the length of the channels defining the zigzag pipe.

The enclosure is suitable for being suspended, so that the direction of the height of said enclosure mainly coincides with the vertical direction and the previously mentioned longitudinal direction mainly coincides with a horizontal direction. Preferably, the enclosure is arranged to be suspended from a horizontal bar supported by a supporting structure.

When the channels are filled with the culture, they preferably form a circular or semi-circular cross section.

The width of the enclosure (the dimension in the direction perpendicular to both the height and length) must preferably be as great as possible, provided that adequate illumination of said enclosure is guaranteed. In this sense, the channel preferably presents a width of between 5 and 20 cm.

The height of the enclosure is preferably limited to two metres, due to considerations relating to the strength of materials and handling the enclosure. Therefore, a length/height ratio of between one and three is preferably recommended to maximise the capacity of the enclosure without incurring excessive tensions in the lower part of this enclosure.

Once the enclosure has been suspended, in the area occupied by the lower part of the enclosure there is a first entry point for introducing the culture coming from the tank through said first entry point; in the area occupied by the upper part of the enclosure, there is a first exit point so that the culture returns to the tank, following a closed circuit.

The design of the photobioreactor of the invention substantially reduces the possibility of contamination because the culture circulates in a closed circuit and therefore reduces the proliferation of other contaminating organisms (such as bacteria and protozoa) that affect the biochemical properties of the cultured phototrophic microorganisms and which could even threaten the survival of these microorganisms, thereby reducing the productivity of the cultured microorganisms.

Another consequence of culture in a closed loop is improved use of the CO₂ provided as a nutrient for the cultured phototrophic microorganisms.

During the circulation of the culture inside the enclosure, the phototrophic microorganisms carry out photosynthesis, assimilating nutrients and releasing oxygen, which dissolves in the culture. The excessive accumulation of oxygen dissolved in the culture must be prevented as it may cause a reduction in the photosynthetic efficiency and, therefore, in the grown of the photoautotrophic microorganisms in question. Addressing this limitation, and because this is a closed culture, limitations in the maximum volume of the enclosure are set in order to avoid excessive accumulation of dissolved oxygen.

The tank is for the storage and homogenisation of the culture as well as for the elimination of excess dissolved oxygen. It is made of a strong material. The tank comprises a second exit point and a second entry point, through which culture can be sent to the enclosure and received from the enclosure respectively.

It also comprises a third exit point, through which the culture is sent to an element for the concentration and collection of biomass, for harvesting the cultured phototrophic microorganisms. The tank is not hermetically sealed, and has at least one tube for releasing accumulated gases to the outside. Some means of impulsion, preferably a pump or alternatively an aspiration system by air using the Venturi effect (airlift) are arranged between the tank and the enclosure to drive the culture from the tank to the enclosure through the first pipes.

The tank is preferably adapted for situating it in the open, although it may function properly when situated inside.

The culture driven from the lower part ascends against gravity through the zigzag pipe defined by the channels. Driving against gravity guarantees complete filling of the enclosure, with optimum occupation of it and the avoidance of bags or bubbles of gases.

The culture is moved by the means of impulsion from the tank to the enclosure at a velocity that must be sufficient to guarantee a turbulent flow of culture through the enclosure, optimising exposure of the culture to light and limiting the adhesion of the photoautotrophic organisms to the sides of the channels, preventing in this way the deposition of the microorganisms on the walls, which would obstruct the passage of light and encourage degradation of the deposited biomass and the eventual proliferation of heterotrophic microorganisms.

Also, the speed is subject to an upper limit, with the object of preventing situations of stress to the cultured photoautotrophic microorganisms that could cause cellular damage to these organisms.

The capacity of the tank and the volume of the culture that it must contain are parameters that depend on various factors, essentially the volume of the enclosure and the speed of flow of the culture, with the need to also guarantee that excess dissolved oxygen is removed during the time of residence of the culture in the tank.

Although the flow of culture that is established in the tank promotes active exchange of gases between the culture, which is a liquid medium, and the air in the tank above the culture, which constitutes the gas phase, there is additionally some means of desorption in the tank in order to homogenise the culture still more and to accelerate the desorption of oxygen dissolved in the culture, facilitating the escape of this dissolved oxygen to the atmosphere in the form of gaseous oxygen.

The means of desorption can be mechanical stirrers arranged inside the tank, which stir the culture using kinetic energy, causing the generation and release of oxygen bubbles. However, more preferably, there may be bubblers arranged in the bottom of the tank because the desorption efficiency of bubblers is still higher.

The tank may additionally include some first means of control to maintain the temperature of the culture within suitable limits for the cultured photoautotrophic microorganisms. These first means of control preferably comprise: a heat exchanger to supply or remove heat from the tank, at least a first temperature probe to capture temperature values of the culture at one or several points in the tank, and a first control unit, for receiving the temperature values of the culture from the first probe and to command the operation of the heat exchanger as a function of the temperature values received.

Additionally, the tank includes some means of supply, the purpose of which is to supply the tank with the compounds necessary for the culture of the microorganisms: in this respect, the means of supply comprise:
- a third entry point for the introduction to the tank of the culture medium prepared with water (fresh or salted as appropriate) and essential nutrients dissolved in water and also, if appropriate, an inoculation of photoautotrophic microorganisms to start a new culture, and
- a valve, preferably of solenoid type, arranged in the lower part of the enclosure, for introducing CO₂ into said enclosure.

The CO₂ is an essential nutrient for the culture and can come from commercial containers (bottles of compressed gas) or combustion gases from various sources that emit this greenhouse gas. The injection of CO₂ is controlled by a second means of control that comprise the following components:
- at least a second probe, for pH, connected to the CO₂ valve, and
- a second control unit, which receives the pH values measured in the culture by the second probe and commands, depending on the values of the pH, the operation of the valve.

The photobioreactor of the invention can optionally include a third means of control for controlling the oxygen dissolved in the culture. The third means of control comprises a third probe for oxygen to determine the concentration of oxygen dissolved in the culture; and a third control unit (preferably shared with the first means of control and the second means of control), connected to the means of desorption and to the third probe, and for receiving the values of the concentration of dissolved oxygen from the third probe and to command the operation of the means of desorption, depending on the values of oxygen concentration received.

The various control elements, of temperature, pH and, eventually, dissolved oxygen, can be integrated into the same unit of the control system for the various parameters, which would simplify and reduce the cost of the equipment. This is equivalent to saying that preferably there is only one control unit in common to the first, second and third means of control.

Of particular interest is the arrangement of a plurality of enclosures connected to the same tank. In this way, the installation costs would be significantly reduced, once the tank size is set in accordance with the number of enclosures to which it is connected. The cost savings are reflected, among other aspects, in the savings in the number of tanks, which additionally implies a corresponding saving in space, as well as avoiding the need to duplicate the entry and exit pipes from the tank, and also a saving in simplifying the means of control.

Due to the higher efficiency in the production of photoautotrophicmicroorganisms that the photobioreactor of the invention has over other alternatives, the requirements of mechanical strength of the supporting structure are substantially reduced in comparison to the reactors described in the state of the art. A preferred example of a supporting structure comprises two elements arranged in parallel, each composed of three frame elements, preferably of aluminium. One of them is a first frame element to be arranged on the ground, while the other two are second frame elements to be arranged fixed to the first frame element above the first frame. In turn, the second frame elements are fixed together at a point sufficiently distant from their free ends to enable suspension of the bar that supports the enclosure.

### DESCRIPTION OF THE FIGURES

In order to complement the description given below and with the aim of providing better understanding of the characteristics of the invention, in accordance with a preferred practical example of it, this description is accompanied by a set of figures that form an integral part of it, in which, for the purpose of illustration and without limitation, the following is represented:
Figure 1. Schema of the disposition and operation of the photobioreactor of the invention.
Figure 2. Perspective view of the enclosure incorporated in the photobioreactor of the invention, suspended from a supporting structure that comprises triangular frame elements.
Figure 3. Perspective view of the enclosure incorporated in the photobioreactor of the invention, suspended from a supporting structure that comprises quadrangular frame elements.
Figure 4. Shows a perspective view of another type of supporting structure in the form of a T for supporting the photobioreactor enclosure.
Figures 5.1 to 5.4. Show a detail view of the configuration of the channels in one of the angled ends, in which different forms of join by thermosealing of the sheets of plastic material that make up the enclosure can be observed.

### PREFERRED EMBODIMENT OF THE INVENTION

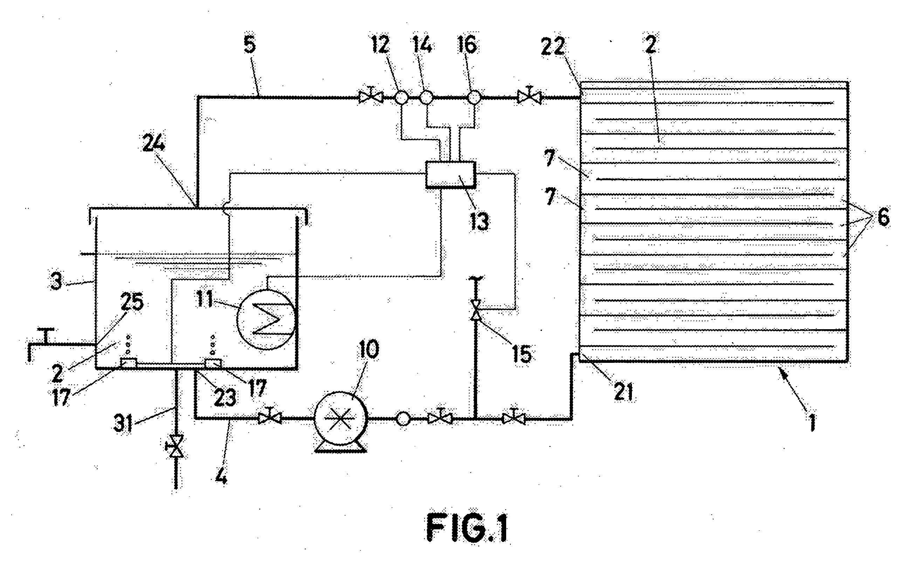

A detailed description of a preferred embodiment of the invention is given below, with reference to figures 1 to 3 attached.

The present invention, as shown in figure 1, is of a photobioreactor for culturing photoautotrophic microorganisms, such as microalgae and/or cyanobacteria, that comprises an enclosure (1) for the culture, adapted for the circulation of a culture (2) of photoautotrophic microorganisms, and which enables access of light to the culture (2), and a tank (3) connected to said enclosure (1) by means of first pipes (4) adapted to conduct the culture (2) from the tank (3) to the enclosure (1), as well as second pipes (5), adapted to conduct the return to the tank (3) of the culture (2) from the enclosure (1), in a closed circuit.

The enclosure (1) is suitable for leaving out in the open, so that the culture (2) that circulates within the enclosure (1) is exposed to sunlight. For this, the enclosure (1) has the properties of mechanical strength, light transmission, resistance to solar degradation and chemical attack, as well as being thermal insulation, which are suitable for being left out in the open. Said enclosure (1) is made from two sheets of low density polyethylene, fixed together by bands of thermosealant that define a plurality of parallel channels (6) suitable for holding the culture (2), with these channels (6) having openings (7) at alternate ends, so that each channel (6) is connected to the contiguous channel (6) or channels (6), forming a single zigzag pipe through which the culture (2) circulates.

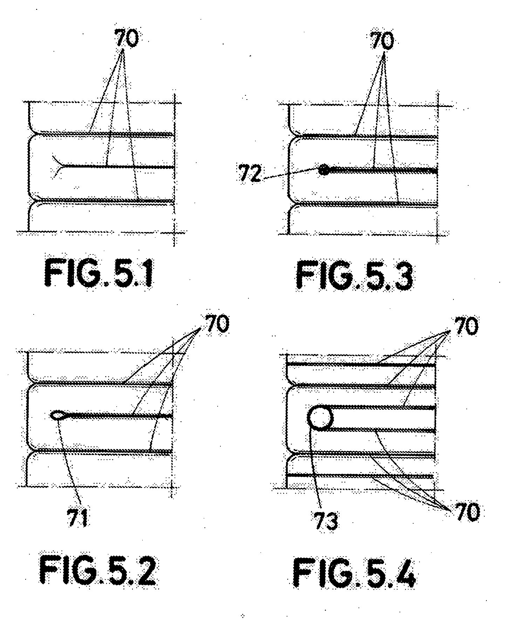

The bands of thermosealant are distributed in parallel in the enclosure (1) to define the indicated zigzag form of the channels (6). It is envisaged that the enclosure (1) adopts a particular configuration of thermosealant bands that are reproduced throughout the enclosure, conformed by a section that extends from one side of the enclosure and terminates at a free end close to the opposing side of the enclosure. Figures 5.1 to 5.4 show different configurations of thermosealant bands with a particular design that favours robustness and strength of the formed enclosure.

In the embodiment shown in figure 5.1, it is envisaged that each of the thermosealant bands comprises a straight section (70).

In the embodiment shown in figure 5.2, each of the thermosealant bands comprises a straight section (70) that has an annular bifurcation in the form of a drop (71) at the free end. In the embodiment shown in figure 5.3, each of the thermosealant bands comprises a straight section (70) that has a circular widening (72) at the free end. In the embodiment shown in figure 5.4, each of the thermosealant bands comprises a pair of straight parallel sections (70) that have a circular ring (73) that links both sections at the free end.

In the following, the length of the enclosure (1) will be considered to be the dimension of the longitudinal direction, that is the direction of the channels (6). The channels (6) present a form such that, when full of culture (2), they preferably form a circular or semi-circular cross section. The width of the channels (6) is between 5 and 20 cm.

Due to the strength of the materials and the handling of the enclosure (1), the height of the enclosure (1) is limited to two metres, being similarly recommended that the length/height ratio is between one and three.

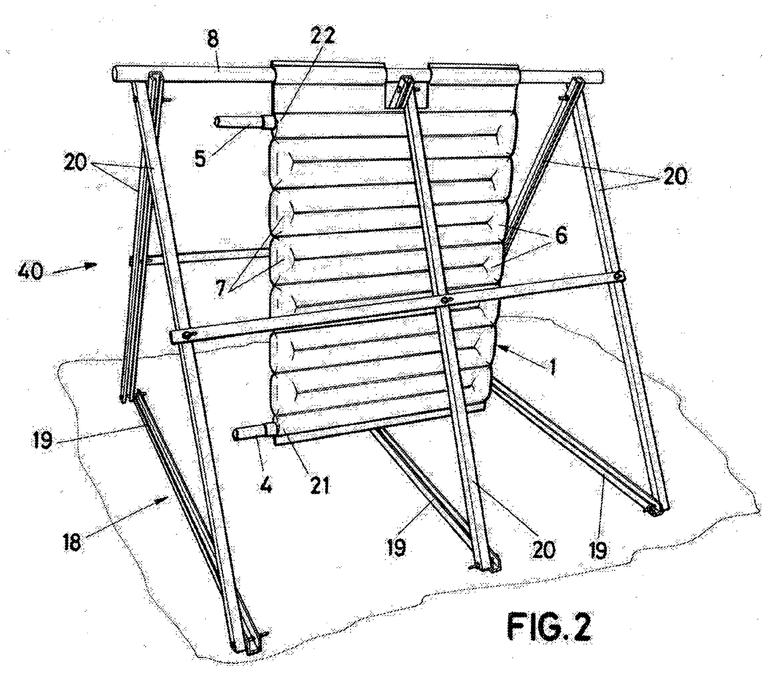

The enclosure (1) is suspended from a horizontal bar (8) supported by a supporting structure (40, 50, 60) (see figures 2, 3 and 4) in such a way that the direction of the height of said enclosure (1) substantially coincides with the vertical direction and the previously mentioned longitudinal direction substantially coincides with a horizontal direction.

We wish to highlight that any supporting structure may be adequate that enables supporting at least one bar (8), from which bar(s) (8) one or more than one enclosures (1) can be suspended. However, some examples will be described below that merely illustrate preferred embodiments of said structure based on their simplicity and low price.

In the lower area of the enclosure (1) there is a first entry point (21) adapted to introduce the culture (2) from the tank (3); similarly in the top of the enclosure (1) there is a first exit point (22) adapted to remove the culture (2) and return it to the tank (3), in a closed circuit.

The tank (3), also suitable in its properties to be left out in the open, is for keeping and homogenising the culture (2). It is made of a strong material such as PVC or other materials in order to reduce costs. The tank comprises a second exit point (23) and a second entry point (24), through which culture (2) can be sent to the enclosure (1) and said culture (2) received from the enclosure (1). It also comprises a third exit point (25) for harvesting the cultivated photoautotrophic microorganisms. Some means of impulsion (10), preferably a pump (10) or alternatively an aspiration system (not shown) by air using the Venturi effect (airlift) are arranged between the tank (3) and the enclosure (1) to drive the culture (2) from the tank (3) to the enclosure (1) through the first pipes (4).

The culture (2) is driven, by the means of impulsion (10), from the lower part of the enclosure (1), ascending against gravity through the zigzag pipe defined by the channels (6).

In order to homogenise the culture, within the tank (3) are some means of desorption (17), to accelerate the desorption of the oxygen dissolved in the culture, facilitating the escape of said dissolved oxygen into the atmosphere in the form of gaseous oxygen. The means of desorption (17) are bubblers (17) arranged in the bottom of the tank (3).

The tank (3) additionally incorporates some first means of control (11, 12, 13) to maintain the temperature of the culture (2) in the tank (3) within levels suitable for the species of cultivated photoautotrophic microorganisms. The first means of control (11, 12, 13) comprise a heat exchanger (11) to provide or remove heat from the tank (3), at least a first probe (12) to capture temperature values of the culture (2) at one or several points of the tank (3), and a control unit (13) for receiving the temperature values of the culture (2) from the first probe (12) and command the operation of the heat exchanger (11) depending on the received temperature values.

Additionally, the tank (3) incorporates some means of supply (31, 15) to provide the compounds necessary for culturing the photoautotrophic microorganisms: said means of supply (31, 15) comprise:
- a third entry point (31) to supply the tank (3) with water (fresh or salted, as appropriate), macro and micronutrients and, if appropriate, an inoculation of photoautotrophic microorganisms to start the culture (2); and
- a solenoid valve (15) to provide CO₂ to the lower part of the enclosure (1).

The CO₂ can come from either commercial containers (bottles) or from combustion fumes, for example, from companies obtaining energy by the combustion of fossil fuels. The supply of CO₂ is controlled by means of a second automated means of control (13, 14), comprising at least a second probe (14) placed at the exit point of the upper part of the enclosure (1) and connected to a valve (15) for the addition of CO₂ and to a control unit (13); said control unit (13) is additionally adapted to receive the values of the pH measured in the culture (2) by the second probe (14) and to command the operation of the valve (15) for the addition of CO₂ depending on the values of the pH received.

The photobioreactor of the invention optionally includes a third means of control (13, 16, 17) for controlling the oxygen dissolved in the culture (2). The third means of control (13, 16, 17) comprise at least a third probe (16), arranged at least at the exit point of the upper part of the enclosure (1) to determine the concentration of dissolved oxygen in the culture (2); and a third control unit (13) connected to the means of desorption (17) and the third probe (16), said control unit (13) is additionally adapted to receive the values of the concentration of dissolved oxygen from the third probe (16) and to command the operation of the means of desorption (17) depending on said values of received oxygen concentration.

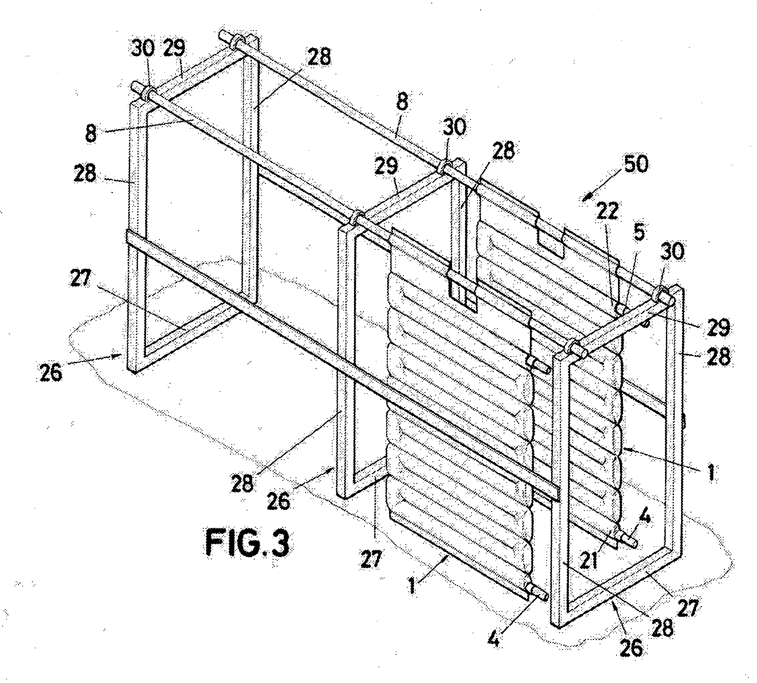

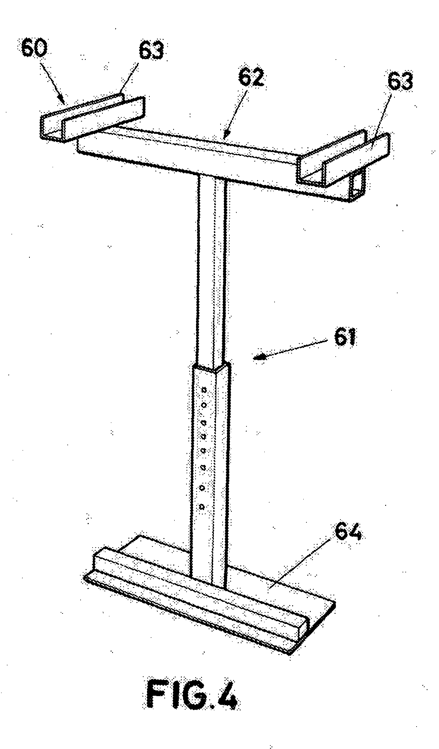

As shown in figures 2 and 3, the supporting structures (40, 50) preferably comprise two frame elements (18, 26) in the form of a polygon arranged in parallel, and each composed of aluminium profiles (19, 20, 27, 28, 29). In another alternative embodiment of the supporting structure (60), shown in figure 4, an element in the form of a T is incorporated, where the vertical arm can be regulated in height.

In accordance with the first example of the supporting structure (40), as shown in figure 2, some first triangular elements (18) comprise a first profile (19) on the ground and two second profiles (20) fixed to the first profile (19) above said first profile (19). In turn, the second profiles (20) are fixed together at a point sufficiently distant from their free ends to enable support of the bar (8) for suspending the enclosure (1).

In accordance with another example of the supporting structure (50) as shown in figure 3, some second quadrangular elements (26) comprise a third profile (27) on the ground, two fourth profiles (28) fixed to each end of the third profile (27) and above said third profile (27) a fifth profile (29) fixed at its ends to the fourth profiles (28) so that on the fifth profile (29) can be fixed, using suitable means of fixing (30), at least one bar (8), to suspend the enclosure (1) from this bar (8). Preferably, two bars (8) would be fixed to take better advantage of the space.

There are no drawbacks to using polygonal elements (18, 26) of more than four sides, although those of three and to a greater extent those of four are preferable. There are also no drawbacks in using more than two bars (8) in the second profiles (20) and in the fifth profiles (29). Other variants such as providing feet (not shown) in the first profiles (19) or in the third profiles (27) are also possible.

The supporting structure (60) shown in figure 4 has a T-shaped configuration formed by a vertical arm (61) topped by a horizontal arm (62) that is extended on both sides and the ends of which terminate in gutters (63) that make up the supporting surfaces for the bars (8) of the enclosures that are suspended on both sides. The vertical arm (61) is adjustable in height, preferably telescopically, as can be seen in figure 4. The supporting structure (60) can have a base (64) from which the vertical arm (61) extends.

## Claims

1. Photobioreactor for culturing photoautotrophic microorganisms, comprising:
- at least one enclosure (1) for performing a culture (2) of photoautotrophic microorganisms, the enclosure (1) being adapted so that the culture (2) can flow through it, the enclosure (1) enabling access of light to the culture (2), and the enclosure (1) comprising:
- two sheets of flexible polymeric plastic material, which are transparent or translucent,
- thermosealant bands comprised in the sheets for fixing the sheets together, defining a plurality of parallel channels (6) for holding the culture (2),
- openings (7) located at alternative ends of the channels (6), so that each channel (6) connects to the contiguous channel (6) or channels (6), forming a single zigzag pipe for the flow of the culture (2), where the enclosure (1) is adapted to being suspended;
- a tank (3) for storing and homogenising the culture (2);
- first pipes (4) for connecting the tank (3) to the bottom of the enclosure
- (1); and
- second pipes (5) for conducting the return of the culture (2) from the top of the enclosure (1) to the top of the tank (3) in a closed circuit;
**characterised in that** it additionally comprises means of impulsion (10), set between the tank (3) and the enclosure (1) to drive the culture (2) from the bottom of the tank (3) to the bottom of the enclosure (1) through the first pipes (4), so as to impulse the culture (2) through the enclosure (1) against gravity.

2. Photobioreactor for culturing photoautotrophic microorganisms according to any of claim 1 **characterised in that** the channels (6) have a width comprised between 5 and 20 cm and the channels (6) have a form that when full of culture (2) present a circular or semi-circular cross section.

3. Photobioreactor for culturing photoautotrophic microorganisms according to claim 1 **characterised in that** the enclosure (1) has a height of not greater than two metres.

4. Photobioreactor for culturing photoautotrophic microorganisms according to any of claims 1 and 3 **characterised in that** the enclosure (1) has a length/height ratio of between one and three.

5. Photobioreactor for culturing photoautotrophic microorganisms according to claim 1 **characterised in that** the thermosealant bands are distributed in parallel in the enclosure (1) to define channels (6) in zigzag form, wherein each thermosealant band comprises at least one section that extends from a side of the enclosure and terminates at a free end close to the opposite side of the enclosure (1).

6. Photobioreactor for culturing photoautotrophic microorganisms according to claim 5 **characterised in that** the section is a straight section (70) and it hast at its free end either an annular bifurcation in the form of a drop (71) or it terminates in a circular widening (72).

7. Photobioreactor for culturing photoautotrophic microorganisms according to claim 5 **characterised in that** it comprises a pair of straight parallel sections (70) that terminate tangentially at their free ends in a circular annular configuration (73) that links both sections.

8. Photobioreactor for culturing photoautotrophic microorganisms according to claim 1 **characterised in that** the tank (3) additionally incorporates first means of control (11,12,13) to maintain the temperature of the culture (2) in the tank (3) within temperature levels suitable for the species of cultivated photoautotrophic microorganisms.

9. Photobioreactor for culturing photoautotrophic microorganisms according to claim 8 **characterised in that** the first means of control (11,12,13) comprise:
- a heat exchanger (11) to supply or remove heat from the tank (3);
- at least a first probe (12) to capture values of temperature of the culture (2) at one or several points of the tank (3); and
- a first control unit (13) for receiving the values of temperature of the culture (2) from the temperature probe and commanding the operation of the heat exchanger (11) depending on said values of temperature received.

10. Photobioreactor for culturing photoautotrophic microorganisms according to claim 1 **characterised in that** the tank (3) additionally incorporates means of supply (31, 15) to provide the compounds necessary for cultivating photoautotrophic microorganisms, said means of supply (31, 15) comprising:
- a third entry point (31) to supply the tank (3) with water (fresh or salted, as appropriate), macro and micronutrients and, if appropriate, an inoculation of photoautotrophic microorganisms to start the culture (2); and
- a solenoid valve (15) to provide C02 to the lower part of the enclosure (1),
where the photobioreactor additionally incorporates some second means of control (13, 14) comprising at least a second probe (14) connected to the valve (15) for addition of C02 and a second control unit (13), said second control unit (13) is adapted for receiving the values of pH measured in the culture (2) by the second probe (14) and commanding the addition of C02 from the valve (15) depending on the said values of pH received.

11. Photobioreactor for culturing photoautotrophic microorganisms according to claim 1 **characterised in that** the tank (3) also incorporates:
- third means of control (13, 16, 17) for controlling the oxygen dissolved in the culture (2) comprising at least a third probe (16) for determining the concentration of oxygen dissolved in the culture (2);
- means of desorption (17) for desorbing oxygen dissolved in the culture (2) in the tank (3), causing the transformation of dissolved oxygen into oxygen bubbles; and
- a third control unit (13) connected to the means of desorption (17) and to the third probe (16), for receiving the values of concentration of dissolved oxygen from the third probe (16) and commanding the operation of the means of desorption (17) depending on said values of oxygen concentration received.

12. Photobioreactor for culturing photoautotrophic microorganisms according to claim 11 **characterised in that** the means of desorption (17) are bubblers (17) arranged in the bottom of the tank (3).

13. Photobioreactor for culturing photoautotrophic microorganisms according to claims 9, 10 and 11 **characterised in that** the control unit (13) is common to the first means of control (11, 12, 13), to the second means of control (13, 14) and the third means of control (13, 16, 17).

14. Photobioreactor for culturing photoautotrophic microorganisms according to claim 1 **characterised in that** il additionally incorporates a supporting structure (40, 50, 60) for supporting a bar (8) from which the enclosure (1) is suspended.

15. Photobioreactor for culturing photoautotrophic microorganisms according to claim 14 **characterised in that** the supporting structure (40, 50) comprises two polygonal elements (18, 26) arranged in parallel, each composed of profiles (19, 20, 27, 28, 29), where one of the profiles (19, 27) is set on the ground, whereas the other profiles (20, 29) are arranged lo support at least one bar (8), two of the profiles (20) are second profiles (20) fixed to the first profile (19) and above said first profile (19), and the second profiles (20) are fixed together at a point sufficiently far from the free ends to allow supporting the bar (8).

16. Photobioreactor for culturing photoautotrophic microorganisms according to claim 14 **characterised in that** the supporting structure (60) has a T-shaped configuration formed by a vertical arm (61) topped by a horizontal arm (62) that is extended on both sides and the ends of which terminate in gutters (63) that make up the supporting surfaces for the bars (8) of the enclosures that are suspended on both sides.

## Patentansprüche

1. Photobioreaktor zur Kultur photoautotropher Mikroorganismen, umfassend:
- mindestens ein Gehäuse (1) zur Durchführung einer Kultur (2) photoautotropher Mikroorganismen, wobei das Gehäuse (1) so ausgelegt ist, dass die Kultur (2) durch dieses fließen kann, wobei das Gehäuse (1) den Zugang von Licht zur Kultur (2) ermöglicht und Folgendes umfasst:
- zwei Folien eines polymeren Kunststoffmaterials, die durchsichtig oder durchscheinen sind;
- in den Folien enthaltene wärmeabdichtende Bänder, um die Folien miteinander zu verbinden, die eine Vielzahl paralleler Kanäle (6) definieren, um die Kultur (2) zu halten;
- Öffnungen (7) an alternativen Enden der Kanäle (6), sodass jeder Kanal (6) mit dem angrenzenden Kanal (6) oder den angrenzenden Kanälen (6) verbunden ist und ein einziges Zickzackrohr für den Durchfluss der Kultur (2) bildet, wobei das Gehäuse (1) dazu ausgelegt ist, aufgehängt zu werden;
- einen Tank (3) zur Lagerung und Homogenisierung der Kultur (2);
- erste Rohre (4) zur Verbindung des Tanks (3) mit dem Boden des Gehäuses (1); und
- zweite Rohre (5) zur Rückleitung der Kultur (2) von der Oberseite des Gehäuses (1) zur Oberseite des Tanks (3) in einem geschlossenen Kreislauf;
**dadurch gekennzeichnet, dass** dieser zusätzlich Antriebsmittel (10) umfasst, die zwischen dem Tank (3) und dem Gehäuse (1) vorgesehen sind, um die Kultur (2) durch die ersten Rohre (4) vom Boden des Tanks (3) zum Boden des Gehäuses (2) zu treiben, damit die Kultur (2) entgegen der Schwerkraft durch das Gehäuse (1) gefördert wird.

2. Photobioreaktor zur Kultur photoautotropher Mikroorganismen nach Anspruch 1, **dadurch gekennzeichnet, dass** die Kanäle (6) eine Breite zwischen 5 und 20 cm besitzen und dass die Kanäle (6) eine Form besitzen, die einen kreisförmigen oder halbkreisförmigen Querschnitt aufweist, wenn diese mit der Kultur (2) gefüllt sind.

3. Photobioreaktor zur Kultur photoautotropher Mikroorganismen nach Anspruch 1, **dadurch gekennzeichnet, dass** das Gehäuse (1) eine Höhe nicht über zwei Meter besitzt.

4. Photobioreaktor zur Kultur photoautotropher Mikroorganismen nach jedem der Ansprüche 1 und 3, **dadurch gekennzeichnet, dass** das Gehäuse (1) ein Längen/Höhenverhältnis zwischen eins und drei besitzt.

5. Photobioreaktor zur Kultur photoautotropher Mikroorganismen nach Anspruch 1, **dadurch gekennzeichnet, dass** die wärmeabdichtenden Bänder parallel im Gehäuse (1) verteilt sind, um Kanäle (6) in Zickzackform zu definieren, wobei jedes wärmeabdichtende Band mindestens einen Abschnitt umfasst, der sich über eine Seite des Gehäuses erstreckt und an einem freien Ende in der Nähe der gegenüberliegenden Seite des Gehäuses (1) endet.

6. Photobioreaktor zur Kultur photoautotropher Mikroorganismen nach Anspruch 5, **dadurch gekennzeichnet, dass** der Abschnitt ein gerader Abschnitt (70) ist und an seinem freien Ende entweder eine ringförmige Gabelung in Form eines Tropfens (71) besitzt oder in einer kreisförmigen Verbreiterung (72) endet.

7. Photobioreaktor zur Kultur photoautotropher Mikroorganismen nach Anspruch 5, **dadurch gekennzeichnet, dass** dieser ein Paar gerader, paralleler Abschnitte (70) umfasst, die an ihren freien Enden tangential in einer kreisförmig ringförmigen Konfiguration (73) enden, die beide Abschnitte verknüpft.

8. Photobioreaktor zur Kultur photoautotropher Mikroorganismen nach Anspruch 1, **dadurch gekennzeichnet, dass** der Tank (3) zusätzlich erste Steuermittel (11, 12, 13) umfasst, um die Temperatur der Kultur (2) im Tank (3) innerhalb eines Temperaturbereichs zu halten, der für die Art der kultivierten photoautotrophen Mikroorganismen geeignet ist.

9. Photobioreaktor zur Kultur photoautotropher Mikroorganismen nach Anspruch 8, **dadurch gekennzeichnet, dass** die ersten Steuermittel (11, 12, 13) Folgendes umfassen:
- einen Wärmeaustauscher (11), um den Tank (3) mit Wärme zu versorgen oder Wärme aus diesem zu entfernen;
- mindestens einen ersten Fühler (12) zur Erfassung von Temperaturwerten der Kultur (2) an einer Stelle oder mehreren Stellen des Tanks (3); und
- eine erste Steuereinheit (13) zum Empfang der Temperaturwerte der Kultur (2) seitens des Temperaturfühlers und zur Steuerung des Betriebs des Wärmeaustauschers (11) abhängig von diesen empfangenen Temperaturwerten.

10. Photobioreaktor zur Kultur photoautotropher Mikroorganismen nach Anspruch 1, **dadurch gekennzeichnet, dass** der Tank (3) zusätzlich Versorgungsmittel (31, 15) umfasst, um die für die Kultur photoautotoropher Mikroorganismen erforderlichen Verbindungen bereitzustellen, wobei diese Versorgungsmittel (31, 15) Folgendes umfassen:
- eine dritte Eingangsstelle (31) zur Versorgung des Tanks (3) mit Wasser (Frisch- oder Salzwasser, wie erforderlich), Makro- und Mikronährstoffen und, falls angemessen, einer Inokulation photoautotropher Mikroorganismen zum Start der Kultur (2); und
- ein Magnetventil (15), um den unteren Teil des Gehäuses (1) mit CO2 zu versorgen,
wobei der Photobioreaktor zusätzlich einige zweite Steuermittel (13, 14) einschließt, die mindestens einen zweiten Fühler (14) umfassen, der mit dem Ventil (15) zur Hinzufügung von CO2 und einer zweiten Steuereinheit (13) verbunden ist, wobei diese zweite Steuereinheit (13) dazu ausgelegt ist, die vom zweiten Fühler (14) in der Kultur (2) gemessenen pH-Werte zu empfangen und abhängig von diesen empfangenen pH-Werten die Hinzufügung von CO2 seitens des Ventils (15) anzuordnen.

11. Photobioreaktor zur Kultur photoautotropher Mikroorganismen nach Anspruch 1, **dadurch gekennzeichnet, dass** der Tank (3) ebenfalls einschließt:
- dritte Steuermittel (13, 16, 17) zur Steuerung des in der Kultur (2) gelösten Sauerstoffs, die mindestens einen dritten Fühler (16) zur Bestimmung der in der Kultur (2) gelösten Sauerstoffkonzentration umfassen;
- Desorptionsmittel (17) zur Desorption des in der Kultur (2) im Tank (3) gelösten Sauerstoffs, wobei de Umwandlung des gelösten Sauerstoffs in Sauerstoffblasen verursacht wird; und
- eine dritte Steuereinheit (13), die mit den Desorptionsmitteln (17) und dem dritten Fühler (16) verbunden ist, um die Werte der gelösten Sauerstoffkonzentration seitens des dritten Fühlers (16) zu empfangen und abhängig von diesen empfangenen Sauerstoffkonzentrationswerten den Betrieb der Desorptionsmittel (17) anzuordnen.

12. Photobioreaktor zur Kultur photoautotropher Mikroorganismen nach Anspruch 11, **dadurch gekennzeichnet, dass** die Desorptionsmittel (17) am Boden des Tanks (3) angeordnete Bubbler-Systeme (17) sind.

13. Photobioreaktor zur Kultur photoautotropher Mikroorganismen nach einem der Ansprüche 9, 10 und 11, **dadurch gekennzeichnet, dass** die Steuereinheit (13) den ersten Steuermitteln (11, 12, 13), den zweiten Steuermitteln (13,1 4) und den dritten Steuermitteln (13, 16, 17) gemeinsam ist.

14. Photobioreaktor zur Kultur photoautotropher Mikroorganismen nach Anspruch 1, **dadurch gekennzeichnet, dass** dieser zusätzlich eine Stützstruktur (40, 50, 60) zur Abstützung einer Stange (8) einschließt, an der das Gehäuse (1) aufgehängt wird.

15. Photobioreaktor zur Kultur photoautotropher Mikroorganismen nach Anspruch 14, **dadurch gekennzeichnet, dass** die Stützstruktur (40, 50) zwei polygonale, parallel angeordnete Elemente (18, 26) umfasst, die jeweils aus Profilen (19, 20, 27, 28, 29) bestehen, wobei eines der Profile (19, 27) auf dem Boden sitzt, während die anderen Profile (20, 29) so angeordnet sind, dass sie mindestens eine Stange (8) stützen, wobei zwei der Profile (20) am ersten Profil (19) und über diesem ersten Profil (19) befestigte zweite Profile (20) sind, und wobei die zweiten Profile (20) an einem Punkt miteinander verbunden sind, der ausreichend weit von den freien Enden entfernt ist, um eine Abstützung der Stange (8) zu erlauben.

16. Photobioreaktor zur Kultur photoautotropher Mikroorganismen nach Anspruch 14, **dadurch gekennzeichnet, dass** die Stützstruktur (60) eine T-förmige Ausführung besitzt, die von einem vertikalen Arm (61) gebildet wird, über dem sich ein horizontaler Arm (62) befindet, der an beiden Enden herausragt und dessen Enden in Rinnen (63) enden, welche die Stützflächen der Stangen (8) der Gehäuse bilden, die an beiden Seiten aufgehängt sind.

## Revendications

1. Photobioréacteur destiné à la culture de microorganismes photoautotrophes, comprenant :
- au moins une enceinte (1) pour réaliser une culture (2) de microorganismes photoautotrophes, l'enceinte (1) étant adaptée pour que la culture (2) puisse s'écouler à travers elle, l'enceinte (1) permettant à la lumière d'accéder à la culture (2), et l'enceinte (1) comprenant :
∘ deux feuilles en matière plastique polymère flexible, qui sont transparentes ou translucides,
∘ des rubans thermocollants compris dans les feuilles pour fixer les feuilles ensemble, définissant une pluralité de canaux parallèles (6) pour porter la culture (2),
∘ des ouvertures (7) situées à des extrémités alternées des canaux (6), pour que chaque canal (6) soit raccordé au canal (6) contigu ou aux canaux (6), en formant un unique tuyau en zigzag pour l'écoulement de la culture (2), où l'enceinte (1) est adaptée pour être suspendue ;
- un réservoir (3) pour stocker et homogénéiser la culture (2) ;
- des premiers tuyaux (4) pour raccorder le réservoir (3) au fond de l'enceinte (1) ; et
- des deuxièmes tuyaux (5) pour diriger le retour de la culture (2) depuis le sommet de l'enceinte (1) jusqu'au sommet du réservoir (3) dans un circuit fermé ;
**caractérisé en ce qu'**il comprend en outre des moyens d'impulsion (10), installés entre le réservoir (3) et l'enceinte (1) pour diriger la culture (2) depuis le fond du réservoir (3) jusqu'au fond de l'enceinte (1) à travers les premiers tuyaux (4), de façon à pousser la culture (2) dans l'enceinte (1) contre la gravité.

2. Photobioréacteur destiné à la culture de microorganismes photoautotrophes selon la revendication 1, **caractérisé en ce que** les canaux (6) ont une largeur comprise entre 5 et 20 cm et les canaux (6) ont une forme qui fait que lorsqu'ils sont remplis de culture (2) ils présentent une section transversale circulaire ou semi-circulaire.

3. Photobioréacteur destiné à la culture de microorganismes photoautotrophes selon la revendication 1, **caractérisé en ce que** l'enceinte (1) est d'une hauteur qui n'est pas supérieure à deux mètres.

4. Photobioréacteur destiné à la culture de microorganismes photoautotrophes selon n'importe laquelle des revendications 1 et 3, **caractérisé en ce que** l'enceinte (1) présente un rapport longueur/hauteur compris entre un et trois.

5. Photobioréacteur destiné à la culture de microorganismes photoautotrophes selon la revendication 1, **caractérisé en ce que** les rubans thermocollants sont répartis parallèlement dans l'enceinte (1) pour définir des canaux (6) en forme de zigzag, dans lequel chaque ruban thermocollant comprend au moins une section qui s'étend en partant d'un côté de l'enceinte pour se terminer à une extrémité libre proche du côté opposé de l'enceinte (1).

6. Photobioréacteur destiné à la culture de microorganismes photoautotrophes selon la revendication 5, **caractérisé en ce que** la section est une section droite (70) et que soit elle possède à son extrémité libre une bifurcation annulaire en forme de goutte (71) soit elle se termine en un élargissement circulaire (72).

7. Photobioréacteur destiné à la culture de microorganismes photoautotrophes selon la revendication 5, **caractérisé en ce qu'**il comprend une paire de sections droites parallèles (70) qui se terminent tangentiellement à leurs extrémités libres en une configuration circulaire annulaire (73) qui relie les deux sections.

8. Photobioréacteur destiné à la culture de microorganismes photoautotrophes selon la revendication 1, **caractérisé en ce que** le réservoir (3) comporte en outre des premiers moyens de contrôle (11, 12, 13) pour maintenir la température de la culture (2) dans le réservoir (3) dans une fourchette de niveaux de température adaptés à l'espèce de microorganismes photoautotrophes cultivés.

9. Photobioréacteur destiné à la culture de microorganismes photoautotrophes selon la revendication 8, **caractérisé en ce que** les premiers moyens de contrôle (11, 12, 13) comprennent :
- un échangeur de chaleur (11) pour fournir ou retirer de la chaleur au réservoir (3) ;
- au moins une première sonde (12) pour capter des valeurs de température de la culture (2) en un ou plusieurs points du réservoir (3) ; et
- une première unité de contrôle (13) pour recevoir, de la sonde de température, les valeurs de température de la culture (2) et qui commande le fonctionnement de l'échangeur de chaleur (11) en fonction desdites valeurs de température reçues.

10. Photobioréacteur destiné à la culture de microorganismes photoautotrophes selon la revendication 1, **caractérisé en ce que** le réservoir (3) comporte en outre des moyens d'alimentation (31, 15) pour apporter les composés nécessaires à la culture de microorganismes photoautotrophes, lesdits moyens d'alimentation (31, 15) comprenant :
- un troisième point d'entrée (31) pour apporter au réservoir (3) de l'eau (douce ou salée, comme il convient), des macro et micronutriments et, le cas échéant, une inoculation de microorganismes photoautotrophes pour faire démarrer la culture (2) ; et
- une vanne solénoïde (15) pour apporter du CO₂ à la partie inférieure de l'enceinte (1),
où le photobioréacteur comporte en outre des deuxièmes moyens de contrôle (13, 14) comprenant au moins une deuxième sonde (14) raccordée à la vanne (15) pour l'ajout de CO₂ et une deuxième unité de contrôle (13), ladite deuxième unité de contrôle (13) est apte à recevoir les valeurs de pH mesurées dans la culture (2) par la deuxième sonde (14) et à commander l'ajout de CO₂ depuis la vanne (15) en fonction desdites valeurs de pH reçues.

11. Photobioréacteur destiné à la culture de microorganismes photoautotrophes selon la revendication 1, **caractérisé en ce que** le réservoir (3) comporte également :
- des troisièmes moyens de contrôle (13, 16, 17) pour contrôler l'oxygène dissous dans la culture (2) comprenant au moins une troisième sonde (16) pour déterminer la concentration de l'oxygène dissous dans la culture (2) ;
- des moyens de désorption (17) pour désorber l'oxygène dissous dans la culture (2) dans le réservoir (3), en provoquant la transformation de l'oxygène dissous en bulles d'oxygène ; et
- une troisième unité de contrôle (13) raccordée aux moyens de désorption (17) et à la troisième sonde (16), pour recevoir les valeurs de concentration de l'oxygène dissous de la troisième sonde (16) et commander le fonctionnement des moyens de désorption (17) en fonction desdites valeurs de concentration de l'oxygène reçues.

12. Photobioréacteur destiné à la culture de microorganismes photoautotrophes selon la revendication 11, **caractérisé en ce que** les moyens de désorption (17) sont des barboteurs (17) disposés au fond du réservoir (3).

13. Photobioréacteur destiné à la culture de microorganismes photoautotrophes selon les revendications 9, 10 et 11, **caractérisé en ce que** l'unité de contrôle (13) est commune aux premiers moyens de contrôle (11, 12, 13), aux deuxièmes moyens de contrôle (13, 14) et aux troisièmes moyens de contrôle (13, 16, 17).

14. Photobioréacteur destiné à la culture de microorganismes photoautotrophes selon la revendication 1, **caractérisé en ce qu'**il comporte en outre une structure de support (40, 50, 60) pour soutenir une barre (8) à laquelle l'enceinte (1) est suspendue.

15. Photobioréacteur destiné à la culture de microorganismes photoautotrophes selon la revendication 14, **caractérisé en ce que** la structure de support (40, 50) comprend deux éléments polygonaux (18, 26) disposés en parallèle, chacun composé de profils (19, 20, 27, 28, 29), où l'un des profils (19, 27) est installé sur le sol, alors que les autres profils (20, 29) sont disposés pour soutenir au moins une barre (8), deux des profils (20) sont des seconds profils (20) fixés au premier profil (19) et au-dessus dudit premier profil (19), et les seconds profils (20) sont fixés ensemble en un point suffisamment éloigné des extrémités libres pour permettre le soutien de la barre (8).

16. Photobioréacteur destiné à la culture de microorganismes photoautotrophes selon la revendication 14, **caractérisé en ce que** la structure de support (60) a une configuration en T formée d'un bras vertical (61) surmonté d'un bras horizontal (62) qui s'étend des deux côtés et dont les extrémités se terminent par des larmiers (63) qui constituent les surfaces de support pour les barres (8) des enceintes qui sont suspendues des deux côtés.
